# EUROPEAN PATENT APPLICATION

(11) **EP 4 481 002 A1**
(43) Date of publication of application: **25.12.2024**
(21) Application number: 23756547.8
(22) Date of filing: 01.02.2023
(51) Int. Cl.: C08L 75/06, C08L 53/00, C12Q 1/00

(54) **GLUCOSE DIFFUSION CONTROL LAYER SOLUTION FOR CONTINUOUS BLOOD GLUCOSE-MEASURING BIOSENSOR, GLUCOSE DIFFUSION CONTROL LAYER FOR CONTINUOUS BLOOD GLUCOSE-MEASURING BIOSENSOR COMPRISING SAME, AND CONTINUOUS BLOOD GLUCOSE-MEASURING BIOSENSOR COMPRISING SAME**

(30) Priority: 16.02.2022 KR 20220020018
(71) Applicant: SD Biosensor Inc., Suwon-si, Gyeonggi-do 16690 (KR)
(72) Inventor: BAEK, Jaewook, Yongin-si, Gyeonggi-do 16948 (KR); YU, Gyeongwan, Bucheon-si, Gyeonggi-do 14409 (KR); OH, Indon, Suwon-si, Gyeonggi-do 16324 (KR); KIM, Geunhoe, Seongnam-si, Gyeonggi-do 13428 (KR)
(74) Representative: Treeby, Philip David William
(86) International application number: PCT/KR2023/001456
(87) International publication number: WO 2023/158134

(57) **Abstract**

The present invention relates to a glucose diffusion control layer solution for a continuous blood glucose-measuring biosensor, a glucose diffusion control layer for a continuous blood glucose-measuring biosensor, comprising the same, and a continuous blood glucose-measuring biosensor comprising the same. More specifically, the present invention relates to: a glucose diffusion control layer solution for a continuous blood glucose-measuring biosensor, in which, by using only a substance of which biocompatibility has been verified, stability can be ensured even when injected into the human body, and glucose permeability and water absorbance can be controlled; a glucose diffusion control layer for a continuous blood glucose-measuring biosensor, comprising the same; and a continuous blood glucose-measuring biosensor comprising the same.

## Description

### [Technical Field]

The present invention relates to a glucose diffusion control layer solution for a continuous blood glucose-measuring biosensor, a glucose diffusion control layer for a continuous blood glucose-measuring biosensor including the same, and a continuous blood glucose-measuring biosensor including the same. More specifically, the present invention relates to a glucose diffusion control layer solution for a continuous blood glucose-measuring biosensor, in which, by using only a substance of which biocompatibility has been verified, stability can be ensured even when injected into the human body, and glucose permeability and water absorbance can be controlled; a glucose diffusion control layer for a continuous blood glucose-measuring biosensor including the same; and a continuous blood glucose-measuring biosensor including the same.

### [Background Art]

According to the IDF Diabetes ATLAS 9^{th} Edition, which is a diabetes white paper published by the International Diabetes Federation (IDF) every two years, in 2019, 463 million (9.3%) were diabetic patients aged 20 to 79 worldwide. This is an increase of 39 million compared to the 425 million reported in 2017.

In addition, as of 2019, there were 136 million diabetic patients over the age of 65, and 1 in 5 elderly people suffered from diabetes.

It is noteworthy that the prevalence of diabetes is increasing rapidly every year.

In light of current trends, it is expected that the number of diabetes patients worldwide will reach 578 million (10.2%) in 2030, and the number will reach 700 million (10.9%) in 2045, which is a 51% increase from 2019.

Accordingly, the revised 2021 Guidelines of the American Diabetes Association (ADA) states that a continuous glucose monitoring (CGM) system can be used even during hospitalization and recommends multiple doses of insulin therapy to patients regardless of age or type of diabetes.

In particular, blood glucose management through CGM was more effective when programs such as education training and follow-up were provided together, thereby helping to correct blood glucose patterns and improve glycated hemoglobin in glycemic patients.

The Korean Diabetes Association (KDA) 2021 treatment guidelines recommend the use of a real-time continuous blood glucose-measuring device to control blood sugar levels and lower the risk of hypoglycemia in adults with type 1 diabetes, and stated that adults with type 2 diabetes receiving multiple insulin injection therapy may use a real-time continuous glucose-measuring device to control their blood sugar levels.

Adults with type 2 diabetes who use only oral medications or other forms of insulin treatment other than multiple insulin injections can have real-time continuous blood glucose measurements periodically to control blood sugar.

In addition, it was recommended to use the same as it can improve obstetric outcomes by optimally controlling blood sugar levels while lowering the risk of hypoglycemia in pregnant women with type 1 diabetes through rapid blood sugar measurement.

The sensor of such a continuous blood glucose-measuring device may include a glucose diffusion control layer at the outermost part of the sensor.

The glucose diffusion control layer does not negatively affect the human body when the sensor is inserted into the human body, and prevents a sensor material from being discharged into the human body. In addition, it is possible to control the diffusion of glucose introduced from the human body.

As the person moves, the flow rate of glucose flowing into the outermost part of the sensor through the glucose diffusion control layer may increase. Accordingly, it may be difficult to accurately measure blood glucose.

Therefore, as a result of research to overcome these problems, when a glucose diffusion control layer for a continuous blood glucose-measuring biosensor is prepared by using a glucose diffusion control layer solution including a mixture of a specific hydrophilic material and a specific hydrophobic material, the inventors of the present invention confirmed that stability can be secured even when inserted into the human body by using only materials with proven biocompatibility, and glucose permeability and water absorption can be controlled, thereby completing the present invention.

### [Disclosure]

### [Technical Problem]

The present invention has been devised to solve the above problems, and the problems to be solved by the present invention are to ensure stability even when inserted into the human body by using only materials whose biocompatibility has been verified, and to provide a glucose diffusion control layer solution for a continuous blood glucose-measuring biosensor with controllable glucose permeability and water absorption, a glucose diffusion control layer for a continuous blood glucose-measuring biosensor including the same, and a continuous blood glucose-measuring biosensor including the same.

### [Technical Solution]

In order to solve the above-described problems, the glucose diffusion control layer solution of the present invention includes a hydrophilic material and a hydrophobic material, and specifically, the glucose diffusion control layer solution for a continuous blood glucose-measuring biosensor according to the present invention may include an aliphatic polyester-based thermoplastic polyurethane as the hydrophilic material, and the hydrophobic material may include a styrene block copolymer.

According to a preferred exemplary embodiment of the present invention, the aliphatic polyester-based thermoplastic polyurethane may have a specific gravity of 1.0 to 1.2 g/cm³ according to the ASTM D792 measurement method.

According to a preferred exemplary embodiment of the present invention, the aliphatic polyester-based thermoplastic polyurethane may have a shore hardness of 78 to 88A according to the ASTM D2240 measurement method.

According to a preferred exemplary embodiment of the present invention, the aliphatic polyester-based thermoplastic polyurethane may have a flexural modulus of 2,400 to 3,400 psi according to the ASTM D790 measurement method.

According to a preferred exemplary embodiment of the present invention, the aliphatic polyester-based thermoplastic polyurethane may have an ultimate tensile of 1,700 to 2,700 psi in a dry state and an ultimate tensile of 900 to 1,900 psi in a wet state according to the ASTM D412 measurement method.

According to a preferred exemplary embodiment of the present invention, the aliphatic polyester-based thermoplastic polyurethane may have an ultimate elongation of 540 to 1,540 psi in a dry state and an ultimate elongation of 320 to 920 psi in a wet state according to the ASTM D412 measurement method.

According to a preferred exemplary embodiment of the present invention, the styrene block copolymer may have a specific gravity of 0.9 to 1.1 g/cm³ according to the ISO 1183-1 measurement method.

According to a preferred exemplary embodiment of the present invention, the styrene block copolymer may have a shore hardness of 60 to 80A according to the ISO 48-4 measurement method.

According to a preferred exemplary embodiment of the present invention, the styrene block copolymer may have a tensile strength of 8.2 to 10.2 MPa according to the ISO 37 measurement method.

According to a preferred exemplary embodiment of the present invention, the styrene block copolymer may have an elongation at break of 414 to 614% according to the ISO 37 measurement method.

According to a preferred exemplary embodiment of the present invention, the styrene block copolymer may have a tear strength of 15 to 25 N/mm according to the ISO 34-1 measurement method.

According to a preferred exemplary embodiment of the present invention, the hydrophilic material and hydrophobic material may have a weight ratio of 1:0.1 to 4.

Meanwhile, the glucose diffusion control layer for a continuous blood glucose-measuring biosensor may be prepared by drying the glucose diffusion control layer solution of the present invention.

Furthermore, the continuous blood glucose-measuring biosensor of the present invention may include the glucose diffusion control layer of the present invention.

### [Advantageous Effects]

The glucose diffusion control layer solution for a continuous blood glucose-measuring biosensor according to the present invention, the glucose diffusion control layer for a continuous blood glucose-measuring biosensor including the same, and the continuous blood glucose-measuring biosensor including the same only use materials with proven biocompatibility, and when they are inserted into the human body, it is possible to secure safety, and glucose permeability and water absorption can be controlled.

### [Description of Drawings]

FIG. 1 is the results of determining the intensity of current according to the time of test sensors using the glucose diffusion control layer solutions prepared in Examples 1 to 3, the hydrophilic solution prepared in Preparation Example 1, and the hydrophobic solution prepared in Preparation Example 2, respectively.
FIG. 2 is the results of determining the density of current according to the time of test sensors using the glucose diffusion control layer solution prepared in Example 1 and the glucose diffusion control layer solution prepared in Comparative Example 1, respectively.

### [Modes of the Invention]

Hereinafter, with reference to the accompanying drawings, the exemplary embodiments of the present invention will be described in detail so that those skilled in the art can easily practice the present invention. The present invention may be embodied in many different forms and is not limited to the exemplary embodiments set forth herein. In order to clearly describe the present invention in the drawings, parts that are irrelevant to the description are omitted, and the same reference numerals are assigned to the same or similar components throughout the specification.

The biosensor for continuous glucose measurement of the present invention may include a glucose diffusion control layer for a continuous blood glucose-measuring biosensor according to the present invention. In this case, the glucose diffusion control layer for a continuous glucose-measuring biosensor of the present invention may be prepared by drying a glucose diffusion control layer solution for a continuous glucose-measuring biosensor of the present invention, which will be described below.

The biosensor for continuous blood glucose measurement of the present invention is an electrochemical sensor for measuring blood glucose.

As constitutions of such a continuous blood glucose-measuring biosensor, it may include, for example, an electrode, an insulator, a substrate, an electron transfer mediator, an enzyme layer including an enzyme and a crosslinking agent, a glucose diffusion control layer and the like.

The enzyme layer may include an enzyme which is capable of oxidizing or reducing a liquid biological specimen and an oxidation-reduction polymer. It may be an enzyme layer for an electrochemical biosensor including an electron transfer mediator, and an oxidoreductase is a general term for enzymes that catalyze an oxidation-reduction reaction in the living body. In the present invention, in the case of a target substance to be measured, such as a biosensor, it means an enzyme that reacts with the target substance to be reduced. The reduced enzyme reacts with the electron transfer mediator, and the target substance is quantified by measuring signals such as current changes generated at this time. The oxidoreductase that is usable in the present invention may be at least one selected from the group consisting of various dehydrogenases, oxidases, esterases and the like, and depending on the target substance for redox or detection, an enzyme that uses the target substance as a substrate can be selected and used among enzymes belonging to the enzyme group.

More specifically, the oxidoreductase may be at least one selected from the group consisting of glucose dehydrogenase, glutamate dehydrogenase, glucose oxidase, cholesterol oxidase, cholesterol esterase, lactate oxidase, ascorbic acid oxidase, alcohol oxidase, alcohol dehydrogenase, bilirubin oxidase, uricase and the like.

In addition, the electron transfer mediator may include at least one selected from a metal-containing complex and an organic conducting salt.

It may include group 8 elements such as ruthenium complex, osmium complex and ferricyanide complex as a metal-containing complex, and it may include ferrocene and quinone, a derivative of quinone, methylene blue and the like as an organic conductive salt.

Meanwhile, the oxidoreductase may also include a cofactor that serves to store hydrogen taken away by the oxidoreductase from the target substance to be measured (e.g., the target substance), and for example, it may be at least one selected from the group consisting of flavin adenine dinucleotide (FAD), nicotinamide adenine dinucleotide (NAD), pyrroloquinoline quinone (PQQ) and the like.

In addition, the enzyme layer may further include at least one additive selected from the group consisting of a crosslinking agent, a surfactant, a water-soluble polymer, a quaternary ammonium salt, a fatty acid, a thickener and the like, in order to serve as a dispersing agent for dissolving a reagent, an adhesive for preparing a reagent, a stabilizer for long-term storage and the like.

In the case of the electrode, it may include two types of electrodes, such as a working electrode and a counter electrode, or may include three types of electrodes, such as a working electrode, a counter electrode and a reference electrode. In one embodiment, the biosensor according to the present invention may be an electrochemical biosensor which is manufactured by applying an enzyme solution including an enzyme that is capable of oxidizing and reducing glucose, a cross-linker and an electron transfer mediator to a substrate having at least two, and preferably, two or three electrodes, and then drying the solution. For example, in terms of an electrochemical biosensor, it is possible to provide a planar electrochemical biosensor which is characterized in that a working electrode, a counter electrode and a reference electrode are disposed on the same surface of a substrate, an enzyme layer according to the present invention is laminated on the working electrode, an insulator is laminated on the substrate and electrodes (working electrode, counter electrode, a reference electrode), and a glucose diffusion control layer is laminated on the electrode.

In a specific aspect, the substrate may be made of at least one material selected from the group consisting of polydimethylsiloxane (PDMS), polyimide (PI) and polyethylene terephthalate (PET).

In addition, as the working electrode, an electrode made of carbon, gold, platinum or silver may be used.

Additionally, in the case of an electrochemical biosensor having two electrodes, since a counter electrode also serves as a reference electrode, gold, platinum, silver or silver/silver chloride electrodes may be used as the counter electrode, and in the case of a three-electrode electrochemical biosensor including the reference electrode, a silver/silver chloride electrode may be used as a reference electrode, and a carbon electrode may be used as a counter electrode.

As a non-limiting example, silver chloride or silver may be used in the case of two electrodes because the counter electrode also serves as the reference electrode, and in the case of three electrodes, silver chloride or silver may be used as the reference electrode, and a carbon electrode may be used as the counter electrode.

Meanwhile, the glucose diffusion control layer solution for a continuous blood glucose-measuring biosensor of the present invention includes a hydrophilic material and a hydrophobic material. As described above, since the glucose diffusion control layer solution for a continuous blood glucose-measuring biosensor of the present invention includes both of a hydrophilic material and a hydrophobic material, the glucose permeability control is excellent even in the human body environment, and the glucose sensing sensitivity can be implemented. If the glucose diffusion control layer solution for a continuous blood glucose-measuring biosensor of the present invention includes only a hydrophilic material, there may be a problem in that the performance of the sensor is deteriorated due to excessive permeation of glucose at the outermost periphery of the continuous blood glucose-measuring biosensor. Additionally, if only a hydrophobic material is included, there may be a problem in that glucose is not sensed because glucose is not permeated at the outermost part of the biosensor for continuous glucose measurement.

First of all, an aliphatic polyester-based thermoplastic polyurethane may be included as a hydrophilic material.

Specifically, the aliphatic polyester-based thermoplastic polyurethane may have a specific gravity of 1.0 to 1.2 g/cm³, and preferably, 1.1 to 1.16 g/cm³ according to the ASTM D792 measurement method.

In addition, the aliphatic polyester-based thermoplastic polyurethane may have shore hardness of 78 to 88A, and preferably 81 to 85A, according to the ASTM D2240 measurement method.

In addition, the aliphatic polyester-based thermoplastic polyurethane may have a flexural modulus of 2,400 to 3,400 psi, and preferably, 2,700 to 3,100 psi, according to the ASTM D790 measurement method.

In addition, the aliphatic polyester-based thermoplastic polyurethane may have an ultimate tensile strength of 1,700 to 2,700 psi, and preferably, 2,000 to 2,400 psi, in a dry state according to the ASTM D412 measurement method.

In addition, the aliphatic polyester-based thermoplastic polyurethane may have an ultimate tensile strength of 900 to 1,900 psi, and preferably, 1,200 to 1,600 psi, in a wet state according to the ASTM D412 measurement method.

In addition, the aliphatic polyester-based thermoplastic polyurethane may have an ultimate elongation of 540 to 1,540 psi, and preferably, 840 to 1,240 psi, in a dry state according to the ASTM D412 measurement method.

In addition, the aliphatic polyester-based thermoplastic polyurethane may have an ultimate elongation of 320 to 920 psi, and preferably, 520 to 720 psi, in a wet state according to the ASTM D412 measurement method.

Next, it may include a styrene block copolymer as a hydrophobic material.

Specifically, the styrene block copolymer may have a specific gravity of 0.9 to 1.1 g/cm³, and preferably, 1.0 to 1.05 g/cm³, according to the ISO 1183-1 measurement method.

In addition, the styrene block copolymer may have a shore hardness of 60 to 80A, and preferably, 65 to 75A, according to the ISO 48-4 measurement method.

In addition, the styrene block copolymer may have a tensile strength of 8.2 to 10.2 MPa, and preferably, 8.7 to 9.7 Mpa, according to the ISO 37 measurement method.

In addition, the styrene block copolymer may have an elongation at break of 414 to 614%, and preferably, 464 to 564%, according to the ISO 37 measurement method.

In addition, the styrene block copolymer has a tear strength of 15 to 25 N/mm, and preferably, 18 to 22 N/mm, according to the ISO 34-1 measurement method, and preferably, the ISO 34-1 method B (b) (Graves) measurement method.

Meanwhile, in the glucose diffusion control layer solution for a continuous blood glucose-measuring biosensor of the present invention, the hydrophilic material and the hydrophobic material have a weight ratio of 1:0.1 to 4, preferably, a weight ratio of 1:0.1 to 3, and more preferably, a weight ratio of 1:0.15 to 0.8., and if the weight ratio is less than 1:0.1, an excessive amount of glucose permeates at the outermost part of the continuous blood glucose-measuring biosensor, and there may be a problem in that the performance of the sensor is deteriorated, and if the weight ratio is more than 1:4, there may be a problem in that glucose is not sensed because glucose does not penetrate the outermost part of the continuous blood glucose-measuring biosensor.

In addition, the glucose diffusion control layer solution for a continuous blood glucose-measuring biosensor of the present invention may further include an organic solvent, and the concentration of the glucose diffusion control layer solution for a continuous blood glucose-measuring biosensor of the present invention may be adjusted through the organic solvent. Any organic solvent used in the art can be used for the organic solvent of the present invention, and preferably, it may include at least one selected from the group consisting of chloroform, cyclohexanone, cyclopentanone, dimethylacetamide, dimethylformamide, dioxane, methylene chloride, trichloroethane, tetrahydrofuran, xylene and toluene, and more preferably it may include tetrahydrofuran

Hereinafter, the present invention will be described through the following examples. In this case, the following examples are only presented to illustrate the invention, and the scope of the present invention is not limited by the following examples.

### Preparation Example 1: Preparation of hydrophilic solution

2,000 mg of aliphatic polyester-based thermoplastic polyurethane (HP-93A-100, Lubrizol), which is a hydrophilic material, and 50 mL of tetrahydrofuran, which is an organic solvent, were introduced into a homogenizer and dissolved to prepare a hydrophilic solution with a concentration of 40 mg/mL.

### Preparation Example 2: Preparation of hydrophobic solution

2,000 mg of a styrene block copolymer (TM7APO, KRAIBURG), which is a hydrophobic material, and 50 mL of tetrahydrofuran, which is an organic solvent, were introduced into a homogenizer and dissolved for 30 minutes to prepare a hydrophobic solution with a concentration of 40 mg/mL.

### Experimental Example 1: Cytotoxicity experiment

For each of the hydrophilic solution prepared in Preparation Example 1 and the hydrophobic solution prepared in Preparation Example 2, cytotoxicity was evaluated according to the ISO 10993-5 evaluation method, and as a result, it was confirmed that all of the hydrophilic solution prepared in Preparation Example 1 and the hydrophobic solution prepared in Preparation Example 2 had no cytotoxicity.

### Experimental Example 2: Skin sensitization experiment

For each of the hydrophilic solution prepared in Preparation Example 1 and the hydrophobic solution prepared in Preparation Example 2, skin sensitization was evaluated according to the ISO 10993-10 evaluation method, and as a result, it was confirmed that all of the hydrophilic solution prepared in Preparation Example 1 and the hydrophobic solution prepared in Preparation Example 2 did not cause irritation or skin sensitivity.

### Experimental Example 3: Systemic toxicity experiment

For each of the hydrophilic solution prepared in Preparation Example 1 and the hydrophobic solution prepared in Preparation Example 2, systemic toxicity was evaluated according to the ISO 10993-11 evaluation method, and as a result, It was confirmed that all of the hydrophilic solution prepared in Preparation Example 1 and the hydrophobic solution prepared in Preparation Example 2 did not have systemic toxicity.

### Experimental Example 4: Blood compatibility experiment

For each of the hydrophilic solution prepared in Preparation Example 1 and the hydrophobic solution prepared in Preparation Example 2, blood compatibility was evaluated according to the ISO 10993-4 evaluation method, and as a result, it was confirmed that all of the hydrophilic solution prepared in Preparation Example 1 and the hydrophobic solution prepared in Preparation Example 2 had blood compatibility.

### Example 1: Preparation of glucose diffusion control layer solution for continuous blood glucose-measuring biosensor

16 mL of the hydrophilic solution prepared in Preparation Example 1 and 4 mL of the hydrophobic solution prepared in Preparation Example 2 were introduced into a stirrer and stirred to prepare a glucose diffusion control layer solution having a concentration of 40 mg/mL. In this case, the glucose diffusion control layer solution included aliphatic polyester-based thermoplastic polyurethane (HP-93A-100, Lubrizol) and a styrene block copolymer (TM7APO, KRAIBURG) at a weight ratio of 1:0.25.

### Example 2: Preparation of glucose diffusion control layer solution for continuous blood glucose-measuring biosensor

10 mL of the hydrophilic solution prepared in Preparation Example 1 and 10 mL of the hydrophobic solution prepared in Preparation Example 2 were introduced into a stirrer and stirred to prepare a glucose diffusion control layer solution having a concentration of 40 mg/mL. In this case, the glucose diffusion control layer solution included aliphatic polyester-based thermoplastic polyurethane (HP-93A-100, Lubrizol) and a styrene block copolymer (TM7APO, KRAIBURG) at a weight ratio of 1:1.

### Example 3: Preparation of glucose diffusion control layer solution for continuous blood glucose-measuring biosensor

4 mL of the hydrophilic solution prepared in Preparation Example 1 and 16 mL of the hydrophobic solution prepared in Preparation Example 2 were introduced into a stirrer and stirred to prepare a glucose diffusion control layer solution having a concentration of 40 mg/mL. In this case, the glucose diffusion control layer solution included aliphatic polyester-based thermoplastic polyurethane (HP-93A-100, Lubrizol) and a styrene block copolymer (TM7APO, KRAIBURG) at a weight ratio of 1:4.

### Manufacturing Example 1: Manufacture of test sensor

(1) An enzyme solution was prepared by mixing an electron transfer mediator, an enzyme and a crosslinking agent at a volume ratio of 4 : 4 : 1. In this case, an osmium complex was used as the electron transfer mediator, and glutamate dehydrogenase was used as the enzyme.
(2) A test electrode consisting of a working electrode (using a carbon electrode), a reference electrode (using an AgCl electrode) and a counter electrode (using a carbon electrode) was prepared, and 2.5 µL of the enzyme solution was drop casted onto the working electrode of the test electrode.
(3) The test electrode was dried in a dry oven at 35°C for 30 minutes.
(4) A masking film was attached on the working electrode, reference electrode and counter electrode of the test electrode, and each of the glucose diffusion control layer solutions prepared in Examples 1 to 3, the hydrophilic solution prepared in Preparation Example 1, and the hydrophobic solution prepared in Preparation Example 2 was drop casted on the masking film, and then dried to manufacture a test sensor in which a glucose diffusion control layer was formed.

### Experimental Example 5: Measurement of glucose permeability

A 2 mL PBS solution was added to a glass cell for electrochemical measurement, and each of the test sensors manufactured in Manufacturing Example 1 was supported in the PBS solution.

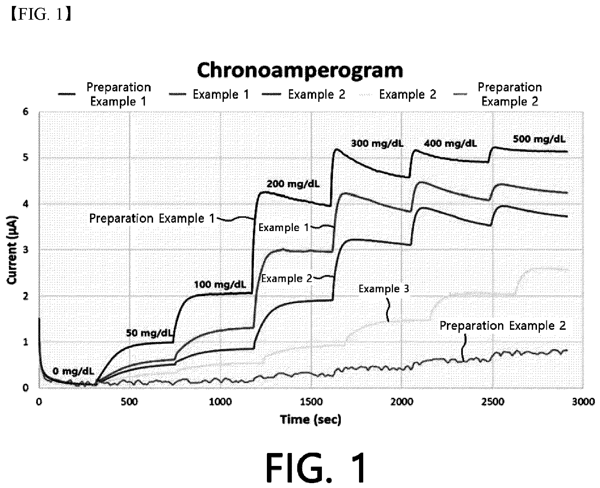

Using a glucose stock solution (in 10 mM PBS solution), glucose was provided to the PBS solution such that the concentration of glucose was 50, 100, 200, 300, 400 and 500 mg/dL, and then, the chronoamperometry (CA) condition was measured through a potentiostat (Multi-Palmsens 4, applied voltage: 0.3 V), and the results are shown in FIG. 1.

As can be confirmed in FIG. 1, it was confirmed that changes in the current value due to changes in the glucose concentration were measured to be large in the order of using the hydrophilic solution prepared in Preparation Example 1, the glucose diffusion control layer solution prepared in Example 1, the glucose diffusion control layer solution prepared in Example 2, and the glucose diffusion control layer solution prepared in Example 3. In addition, when only the hydrophobic solution prepared in Preparation Example 2 was used, there was little change in the current value due to the change in glucose concentration.

Through these results, it was confirmed that the glucose sensing sensitivity can be selectively controlled by controlling the ratio of the hydrophobic material included in the glucose diffusion control layer.

### Experimental Example 6: Measurement of water absorption rate

3 mL of the glucose diffusion control layer solution prepared in Examples 1 to 3, the hydrophilic solution prepared in Preparation Example 1, and the hydrophobic solution prepared in Preparation Example 2 were each dispensed into a glass petri dish, and dried at room temperature (25°C) for 24 hours such that glucose diffusion control layers were prepared, and their initial weights were measured.

The prepared glucose diffusion control layer was immersed in ultrapure water (DI water) for 24 hours, and then, the weight was measured.

Using the measured initial and final weights, the water absorption rate of each of the glucose diffusion control layer solutions prepared in Examples 1 to 3, the hydrophilic solution prepared in Preparation Example 1, and the hydrophobic solution prepared in Preparation Example 2 was calculated, and the results are shown in Table 1 below.

**[Table 1]**

| Classification | Initial Weight (mg) | Final Weight (mg) | Water Absorption Rate (%) |
|---|---|---|---|
| Example 1 | 109.5 | 188.5 | 72.1 |
| Example 2 | 107.6 | 152.0 | 41.3 |
| Example 3 | 109.2 | 123.3 | 12.9 |
| Preparation Example 1 | 106.8 | 2003 | 87.5 |
| Preparation Example 2 | 112.0 | 112.6 | 0.6 |

As can be confirmed in Table 1, it was confirmed that the water absorption rate was measured to be high in the order of using the hydrophilic solution prepared in Preparation Example 1, the glucose diffusion control layer solution prepared in Example 1, the glucose diffusion control layer solution prepared in Example 2, and the glucose diffusion control layer solution prepared in Example 3. In addition, when only the hydrophobic solution prepared in Preparation Example 2 was used, water was hardly absorbed.

Through these results, it was confirmed that the water absorption rate can be selectively controlled by controlling the ratio of the hydrophobic material included in the glucose diffusion control layer.

### Comparative Preparation Example 1: Preparation of hydrophobic solution

2,000 mg of aliphatic polyester-based thermoplastic polyurethane (SG-80A, Lubrizol), which is a hydrophobic material, and 50 mL of tetrahydrofuran, which is an organic solvent, were introduced into a homogenizer and dissolved for 60 minutes to prepare a hydrophobic solution at a concentration of 40 mg/mL.

As can be confirmed in the process of preparing the hydrophobic solution of Comparative Preparation Example 1, it was confirmed that the time taken to dissolve the styrene block copolymer used in Preparation Example 2 was 30 minutes, whereas the time taken to dissolve the aliphatic polyester-based thermoplastic polyurethane used in Comparative Preparation Example 1 was 60 minutes., and through this, when preparing the glucose diffusion control layer solution, it was confirmed that the use of the hydrophobic solution prepared in Preparation Example 2 as a hydrophobic solution shortens the preparation time compared to using the hydrophobic solution prepared in Comparative Preparation Example 1, and thus, it was confirmed that it is easy to mass produce.

### Comparative Example 1: Preparation of glucose diffusion control layer solution for continuous blood glucose-measuring biosensor

16 mL of the hydrophilic solution prepared in Preparation Example 1 and 4 mL of the hydrophobic solution prepared in Comparative Preparation Example 1 were introduced into a stirrer and stirred to prepare a glucose diffusion control layer solution having a concentration of 40 mg/mL. In this case, the glucose diffusion control layer solution included aliphatic polyester-based thermoplastic polyurethane (HP-93A-100, Lubrizol) and aliphatic polyester-based thermoplastic polyurethane (SG-80A, Lubrizol) at a weight ratio of 1:0.25.

### Manufacturing Example 2: Manufacture of test sensor

(1) An enzyme solution was prepared by mixing an electron transfer mediator, an enzyme and a crosslinking agent at a volume ratio of 4 : 4 : 1. I n this case, an osmium complex was used as the electron transfer mediator, and glutamate dehydrogenase was used as the enzyme.
(2) A test electrode consisting of a working electrode (using a carbon electrode), a reference electrode (using an AgCl electrode) and a counter electrode (using a carbon electrode) was prepared, and 2.5 µL of the enzyme solution was drop casted onto the working electrode of the test electrode.
(3) The test electrode was dried in a dry oven at 35°C for 30 minutes.
(4) A masking film was attached on the working electrode, reference electrode and counter electrode of the test electrode, and each of the glucose diffusion control layer solution prepared in Example 1 and the glucose diffusion control layer solution prepared in Comparative Example 1 was drop casted on the masking film and then dried to manufacture a test sensor in which a glucose diffusion control layer was formed, respectively.

### Experimental Example 7: Measurement of glucose permeability

A 2 mL PBS solution was added to a glass cell for electrochemical measurement, and each of the test sensors manufactured in Manufacturing Example 2 was supported in the PBS solution.

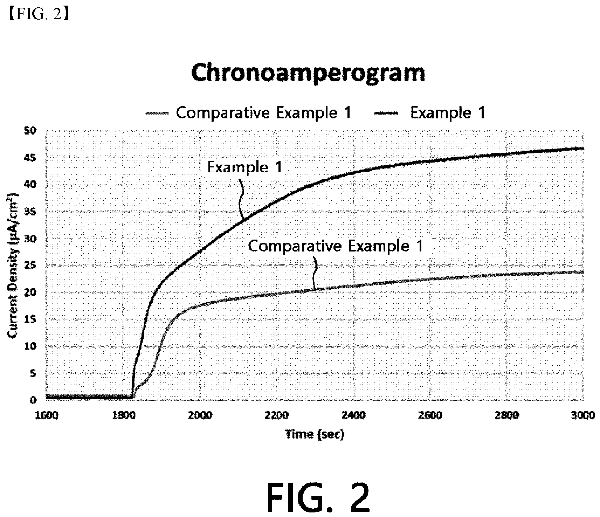

After providing glucose to the PBS solution by using a glucose stock solution (in 10 mM PBS solution) such that the concentration of glucose was 200 mg/dL, the CA (chronoamperometry) condition was measured by using a potentiostat (Multi-Palmsens 4, applied voltage: 0.3 V), and the results are shown in FIG. 2.

As can be confirmed in FIG. 2, when the glucose diffusion control layer solution prepared in Example 1 was used, it was confirmed that the current density was measured to be higher than when the glucose diffusion control layer solution prepared in Comparative Example 1 was used.

Simple modifications or changes of the present invention can be easily performed by those skilled in the art, and all such modifications or changes can be considered to be included in the scope of the present invention.

## Claims

1. A glucose diffusion control layer solution for a continuous blood glucose-measuring biosensor, comprising:
a hydrophilic material; and
a hydrophobic material,
wherein the hydrophilic material comprises an aliphatic polyester-based thermoplastic polyurethane, and
wherein the hydrophobic material comprises a styrene block copolymer.

2. The glucose diffusion control layer solution of claim 1, wherein the aliphatic polyester-based thermoplastic polyurethane has a specific gravity of 1.0 to 1.2 g/cm³ according to the ASTM D792 measurement method, and a shore hardness of 78 to 88A according to the ASTM D2240 measurement method, and
wherein the styrene block copolymer has a specific gravity of 0.9 to 1.1 g/cm³ according to the ISO 1183-1 measurement method, and a shore hardness of 60 to 80A according to the ISO 48-4 measurement method.

3. The glucose diffusion control layer solution of claim 2, wherein the aliphatic polyester-based thermoplastic polyurethane has a flexural modulus of 2,400 to 3,400 psi according to the ASTM D790 measurement method, an ultimate tensile of 1,700 to 2,700 psi in a dry state and an ultimate tensile of 900 to 1,900 psi in a wet state according to the ASTM D412 measurement method, and an ultimate elongation of 540 to 1,540 psi in a dry state and an ultimate elongation of 320 to 920 psi in a wet state according to the ASTM D412 measurement method.

4. The glucose diffusion control layer solution of claim 2, wherein the styrene block copolymer has a tensile strength of 8.2 to 10.2 MPa according to the ISO 37 measurement method, an elongation at break of 414 to 614% according to the ISO 37 measurement method, and a tear strength of 15 to 25 N/mm according to the ISO 34-1 measurement method.

5. The glucose diffusion control layer solution of claim 1, wherein the hydrophilic material and hydrophobic material have a weight ratio of 1:0.1 to 4.

6. A glucose diffusion control layer for a continuous blood glucose-measuring biosensor, which is prepared by drying the glucose diffusion control layer solution of claim 1.

7. A continuous blood glucose-measuring biosensor, comprising the glucose diffusion control layer of claim 6.
